(19) Europäisches Patentamt
European Patent Office
Office européen des brevets

(11) Numéro de publication : **0 433 188 A1**

(12) **DEMANDE DE BREVET EUROPEEN**

(21) Numéro de dépôt : **90420535.8**

(22) Date de dépôt : **11.12.90**

(51) Int. Cl.[5] : **C07D 239/54,** C07D 213/75, C08G 81/02, C07H 19/16, C07H 21/00

(30) Priorité : **13.12.89 FR 8916752**

(43) Date de publication de la demande : **19.06.91 Bulletin 91/25**

(84) Etats contractants désignés : **BE DE FR GB IT NL SE**

(71) Demandeur : **RHONE-POULENC CHIMIE**
**25, quai Paul Doumer**
**F-92408 Courbevoie Cédex (FR)**

(72) Inventeur : **Fouquey, Claudine**
**3, place Paul Painlevé**
**F-75005 Paris (FR)**
Inventeur : **Lehn, Jean-Marie**
**21, rue d'Oslo**
**F-67000 Strasbourg (FR)**

(74) Mandataire : **Trolliet, Maurice et al**
**RHONE-POULENC INTERSERVICES Service Brevets Chimie Centre de Recherches des Carrières B.P. 62**
**F-69192 Saint-Fons Cédex (FR)**

(54) **Associations polymoléculaires éventuellement thermotropes dont les monomères de base sont liés entre eux par des liaisons hydrogène, un procédé servant à les préparer et les monomères de base destinés à la mise en oeuvre de ce procédé.**

(57) La présente invention concerne des associations polymoléculaires, pouvant être thermotropes, dont les monomères de base sont liés entre eux par au moins deux liaisons hydrogène.

Conformément à l'invention, on fait réagir un monomère M1 de formule (Σ

$)_{m1}$A (I) dans laquelle les symboles Σ représentent chacun un groupe fonctionnel capable de former au moins deux liaisons hydrogène avec le groupe fonctionnel complémentaire représenté par chacun des symboles $\bar{\Sigma}$ d'un second monomère M2 de formule ($\bar{\Sigma}$

$)_{m2}$B (II) ; A et B représentent des radicaux organiques de valence m1 et m2 pouvant renfermer jusqu'à 50 atomes de carbone et m1 et m2 représentent des nombres au moins égaux à 2. Comme groupes fonctionnels complémentaires, on citera notamment les couples : cytosine/isocytosine, guanine/cytosine, adénine/uracil, dialkanamido-2,6 pyridine/uracil.

L'invention concerne encore un procédé de préparation desdits polymères et, à titre de produits nouveaux, les monomères de base M1 et M2 destinés à la mise en oeuvre de ce procédé.

EP 0 433 188 A1

# ASSOCIATIONS POLYMOLECULAIRES EVENTUELLEMENT THERMOTROPES DONT LES MONOMERES DE BASE SONT LIES ENTRE EUX PAR DES LIAISONS HYDROGENE, UN PROCEDE SERVANT A LES PREPARER ET LES MONOMERES DE BASE DESTINES A LA MISE EN OEUVRE DE CE PROCEDE

La présente invention, dans son premier objet, concerne des associations polymoléculaires, qui seront définies dans la suite du présent mémoire par l'expression "polymères", dont les monomères de base sont liés entre eux par au moins deux liaisons hydrogène. Ces polymères peuvent être thermotropes si des substitutions appropriées sont introduites sur les monomères de base. Dans un second objet, la présente invention a trait à un procédé de préparation desdits polymères. Dans un troisième objet, elle concerne les monomères de base destinés à la mise en oeuvre de ce procédé mais qui peuvent aussi être utilisés à d'autres fins.

L'art antérieur ne dit rien à propos de la possibilité de réaliser des polymères par une réaction de polyassociation dans laquelle les monomères de base sont liés par des liaisons hydrogène. Le but principal de la présente invention est de proposer un pareil nouveau type de synthèse et de fournir dans ce cadre des polymères dont le niveau de cohésion est suffisamment élevé pour permettre de les utiliser dans certaines applications prévues pour les polymères conventionnels dans lesquels les monomères de base sont liés par des liaisons de covalence.

La présente invention, dans son premier objet, concerne donc des polymères caractérisés en ce qu'ils résultent de l'interaction de :

a) un monomère M1 de formule :

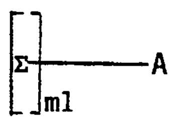

(I)

dans laquelle :

- les symboles Σ, identiques ou différents entre eux, représentent chacun un groupe fonctionnel, dérivant d'une molécule ou d'un groupe de molécules, qui est capable de former au moins deux liaisons hydrogène avec le groupe fonctionnel complémentaire représenté par chacun des symboles $\bar{\Sigma}$ du monomère M2 de formule (II) apparaissant ci-après,
- le symbole A représente un radical organique de valence m1 pouvant renfermer jusqu'à 50 atomes de carbone, et
- m1 représente un nombre au moins égal à 2 et se situant notamment dans l'intervalle allant de 2 à 5 ;

avec b) un monomère complémentaire M2 de formule :

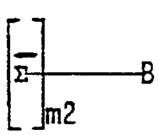

(II)

dans laquelle :

- les symboles $\bar{\Sigma}$, identiques ou différents entre eux, représentent chacun un groupe fonctionnel, dérivant d'une molécule ou d'un groupe de molécules, qui est capable de former au moins deux liaisons hydrogène avec le groupe fonctionnel complémentaire représenté par chacun des symboles Σ du monomère M1 de formule (I) défini ci-avant,
- le symbole B représente un radical organique de valence m2, identique ou différent du radical A, et
- m2 représente un nombre au moins égal à 2 et se situant notamment dans l'intervalle allant de 2 à 5.

Les radicaux A et B dont on vient de parler sont généralement des chaînes hydrocarbonées, linéaires ou ramifiées, saturées ou insaturées, qui comprennent éventuellement un ou plusieurs hétéroatomes.

Une liaison hydrogène est formée habituellement entre un donneur de proton et un accepteur de proton et elle peut être illustrée par exemple

par la formule $>NH \cdots O = C<$ où $>NH$ et $O = C<$ constituent des sites complémentaires.

Les structures des molécules ou groupes de molécules desquels dérivent les groupes fonctionnels complémentaire $\Sigma$ et $\bar{\Sigma}$ ne sont pas particulièrement critiques dès l'instant où :

(i) l'ensemble de ces structures apporte au moins deux paires de sites complémentaires et où les sites complémentaires donneur et accepteur d'une même paire sont capables de se situer sensiblement dans un même plan et de prendre une orientation correcte,

et (2i) lesdites structures permettent l'installation des radicaux espaceurs A et B adéquats.

Comme exemples de sites donneurs et accepteurs, on citera : les sites donneurs $>N-H$, $-NH_2$, $-O-H$ et les sites accepteurs $>C=O$, $-O-$, $=N-$, $>N-$.

Dans le cadre de la présente invention, les groupes fonctionnels $\Sigma$ et $\bar{\Sigma}$, qui doivent comprendre chacun au moins deux sites donneur(s) et/ou accepteur(s), dérivent de molécules ou groupes de molécules consistant généralement dans des composés monocycliques contenant un ou plusieurs hétéroatome(s) tels que notamment N, O, S ou dans des composés polycycliques, ortho- ou péricondensés, contenant également un ou plusieurs hétéroatome(s) tels que notamment N, O, S. Les sites donneurs et accepteurs capables de former des liaisons hydrogène peuvent (i) être contenus dans le (ou les) cycle(s) et/ou (2i) constituer une (ou plusieurs) substitution(s) portée(s) par ce (ou ces) cycle(s).

Lorsque deux monomères complémentaires M1 et M2 sont mis en présence, ils se lient par association de leurs groupes fonctionnels complémentaires $\Sigma$ et $\bar{\Sigma}$ association qui génère dans la structure du polymère des motifs de structure :

$$\text{M1-1}(-)\ A - \Sigma \cdots \bar{\Sigma} - B\ (-)_{m2-1}$$

dans laquelle le symbole " — " compris entre $\Sigma$ et $\bar{\Sigma}$ représentent au moins 2 liaisons hydrogène.

Des exemples de couples de composés cycliques complémentaires, qui peuvent servir à confectionner les monomères de base, puis les motifs d'association $\Sigma$ — $\bar{\Sigma}$ desdits monomères de base conduisant aux polymères selon l'invention, sont notamment : le couple cytosine (ou oxy-2 amino-4 pyrimidine)/isocytosine (ou amino-2 hydroxy-4 pyrimidine) ; le couple guanine (ou amino-2 oxy-6 purine)/cytosine ; le couple adénine (ou amino-6 purine)/uracil (ou dioxy-2,4 pyrimidine) ; le couple dialkanamido-2,6 pyridine/uracil. Conviennent encore les couples différents des couples précités ou leurs analogues qui sont décrits dans les articles suivants :

– FEIBUSH et al., J. Am. Chem. Soc. 1986, 108, 3310-3318, comme par exemple le couple dialkanamido-2,6 pyridine/thymine (ou méthyl-5 dioxy-2,4 pyrimidine), et

– R. KELLY et al., J. Am. Chem. Soc. 1987, 109, 6549-6551.

Dans la présente invention, prise dans son premier objet, on s'intéresse en particulier aux polymères issus de monomères complémentaires M1 et M2 qui sont difonctionnels, c'est-à-dire des monomères de formules (I) et (II) dans lesquelles m1 = m2 = 2. Les symboles A et B représentent alors des radicaux comportant deux valences libres.

De pareils polymères selon l'invention obtenus à partir de monomères difonctionnels peuvent être représentés par la formule générale suivante :

$$\Sigma - A - \Sigma \cdots \left[ \bar{\Sigma} - B - \bar{\Sigma} \cdots \Sigma - A - \Sigma \cdots \right]_n \bar{\Sigma} - B - \bar{\Sigma} \qquad (III)$$

dans laquelle les divers symboles A, B, $\Sigma$, $\bar{\Sigma}$ ont les significations données ci-avant et n représente un nombre entier ou fractionnaire allant par exemple de 0 à 10.

A titre de monomères difonctionnels M1 et M2 qui sont représentatifs des monomères de base utilisables, on peut citer les composés de formules :

$$\underset{M1}{\Sigma - A - \Sigma} \quad (IV) \qquad \text{et} \qquad \underset{M2}{\bar{\Sigma} - B - \bar{\Sigma}} \quad (V)$$

dans lesquelles :

– les groupes fonctionnels complémentaires Σ (identiques ou différents entre eux) et $\overline{\Sigma}$ (identiques ou différents entre eux), qui doivent comprendre chacun au moins deux sites donneur(s) et/ou accepteur(s), dérivent de molécules ou groupes de molécules consistant généralement dans des composés monocycliques contenant un ou plusieurs hétéroatome(s) tels que notamment N, O, S ou dans des composés polycycliques, ortho- ou péricondensés, contenant également un ou plusieurs hétéroatome(s) tels que notamment N, O, S, les sites donneurs et accepteurs capables de former des liaisons hydrogène pouvant (i) être contenus dans le (ou les) cycle(s) et/ou (2i) constituer une (ou plusieurs) substitution(s) portée(s) par ce (ou ces) cycle(s),

– les radicaux espaceurs A et B, identiques ou différents, représentent des radicaux divalents consistant dans des chaînes hydrocarbonées, linéaires ou ramifiées, saturées ou insaturées, comprenant éventuellement un ou plusieurs hétéroatomes tels que notamment N, O, S, qui comportent au moins 2 atomes de carbone dans la chaîne principale, ladite chaîne principale étant celle qui porte en bout de chaîne les deux groupes fonctionnels Σ et $\overline{\Sigma}$.

A titre de monomères difonctionnels M1 et M2 qui sont préférentiellement représentatifs des monomères de base utilisables, on peut citer les composés de formules (IV) et (V) dans lesquelles :

– les groupes fonctionnels complémentaires Σ (identiques ou différents entre eux) et $\overline{\Sigma}$ (identiques ou différents entre eux) dérivent notamment des couples de composés hétérocycliques complémentaires suivants : c1 = cytosine/isocytosine, c2 = guanine/cytosine, c3 = adénine/uracil, c4 = dialkanamido-2,6 pyridine/uracil, c5 = dialkanamido-2,6 pyridine/thymine,

– les radicaux espaceurs A et B sont fixés sur les groupes fonctionnels en des points situés en une position opposée ou sensiblement opposée par rapport à celle occupée par les sites donneur(s) et/ou accepteur(s),

– les radicaux espaceurs A et B, identiques ou différents, représentent des radicaux divalents consistant dans des chaînes hydrocarbonées, linéaires ou ramifiées, saturées ou insaturées, comprenant éventuellement un ou plusieurs hétéroatomes tels que notamment N, O, S, qui comportent au moins 2 atomes de carbone dans la chaîne principale.

A titre de monomères difonctionnels M1 et M2 qui sont plus préférentiellement représentatifs des monomères de base utilisables, on peut citer les composés de formules (IV) et (V) dans lesquelles :

– les paires de groupes fonctionnels complémentaires sont formées chacune de deux groupes Σ identiques et de deux groupes $\overline{\Sigma}$ identiques,

– les groupes fonctionnels complémentaires dérivent notamment des couples de composés hétérocycliques complémentaires suivants : c1 = cytosine/isocytosine, c2 = guanine/cytosine, c3 = adénine/uracil, c4 = dialkanamido-2,6 pyridine/uracil, c5 = dialkanamido-2,6 pyridine/thymine,

– les radicaux espaceurs A et B sont fixés sur les groupes fonctionnels en des points situés en une position opposée ou sensiblement opposée par rapport à celle occupée par les sites donneur(s) et/ou accepteur(s),

– les radicaux espaceurs A et B, identiques ou différents, représentent des radicaux divalents consistant dans des chaînes aliphatiques saturées, linéaires ou ramifiées, comprenant éventuellement un ou plusieurs hétéroatomes tels que notamment N, O, S, qui comportent au moins 6 atomes dans la chaîne principale dont au moins 4 atomes de carbone.

On a constaté que les polymères selon la présente invention, prise dans son premier objet, peuvent être thermotropes si l'on choisit des monomères M1 et M2 difonctionnels :

– ayant les définitions générales ou préférées indiquées ci-avant en ce qui concerne les groupes fonctionnels Σ et $\overline{\Sigma}$ et les points de fixation des bouts de chaîne principale des radicaux espaceurs A et B sur ces groupes fonctionnels,

– mais dans la structure desquels les radicaux espaceurs A et B, identiques ou différents, représentent tous deux des radicaux divalents consistant dans des chaînes ramifiées, comprenant éventuellement un ou plusieurs hétéroatomes, qui répondent aux conditions supplémentaires suivantes :

- la chaîne principle de chaque chaîne ramifiée, qui est celle portant en bout de chaîne les deux groupes fonctionnels Σ et $\overline{\Sigma}$, comporte au moins 6 atomes dont au moins 4 atomes de carbone, et
- la (ou les) chaîne(s) latérale(s) attachée(s) à ladite chaîne principale comporte(nt) au moins 6 atomes dont au moins 5 atomes de carbone.

Il doit être entendu que les atomes qui sont pris en compte dans la détermination du nombre d'atomes sont les atomes de carbone + éventuellement les hétéroatomes qui constituent la chaîne (principale ou latérale) considérée ; par exemple un enchaînement du type : —CH2-O-CO— comporte 3 atomes et un enchaînement du type : —O-CH2-CH2-O-CO— comporte 5 atomes dont 3 atomes de carbone.

A titre de monomères difonctionnels M1 et M2 qui sont encore plus préférentiellement représentatifs des

monomères de base utilisables et qui permettent de préparer en outre des polymères doués de propriétés thermotropes, on peut citer les composés de formule (IV) et (V) dans lesquelles :

– les paires de groupes fonctionnels complémentaires sont formées chacune de deux groupes Σ identiques et de deux groupes $\overline{\Sigma}$ identiques,

– les groupes fonctionnels complémentaires dérivent notamment des couples de composés hétérocycliques complémentaires suivants : c1 = cytosine/isocytosine, c2 = guanine/cytosine, c3 = adénine/uracil, c4 = dialkanamido-2,6 pyridine/uracil, c5 = dialkanamido-2,6 pyridine/thymine,

– les radicaux espaceurs A et B sont fixés sur les groupes fonctionnels en des points situés en une position opposée ou sensiblement opposée par rapport à celle occupée par les sites donneur(s) et/ou accepteur(s),

– les radicaux espaceurs A et B, identiques ou différents, dérivent chacun de l'acide tartrique, pris dans sa configuration méso ou dans ses configurations thréo, et correspondent chacun à la formule :

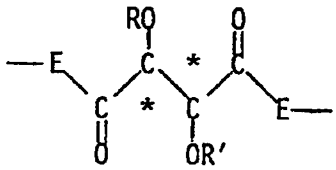

(VI)

dans laquelle :

- les restes E, identiques ou différents, représentent chacun un motif ayant au moins un atome, choisi dans le groupe des atomes de carbone et des hétéroatomes tels que notamment N, O, S, résultant de l'accrochage de la fonction COOH (ou d'un dérivé de cette fonction) de l'acide tartrique sur une fonction réactive appropriée attachée soit directement par un lien de covalence, soit par l'intermédiaire d'un bras d'espacement à base d'atome(s) de carbone et/ou d'hétéroatome(s) sur le groupe fonctionnel Σ ou $\overline{\Sigma}$,
- les symboles R et R', identiques ou différents, représentent chacun un radical alkyle linéaire ayant de 5 à 20 atomes de carbone.

A titre d'exemples spécifiques de monomères M1 et M2 appartenant au groupe des monomères "encore plus préférentiellement représentatifs", on citera notamment les composés de formules :

S'agissant de M1 :

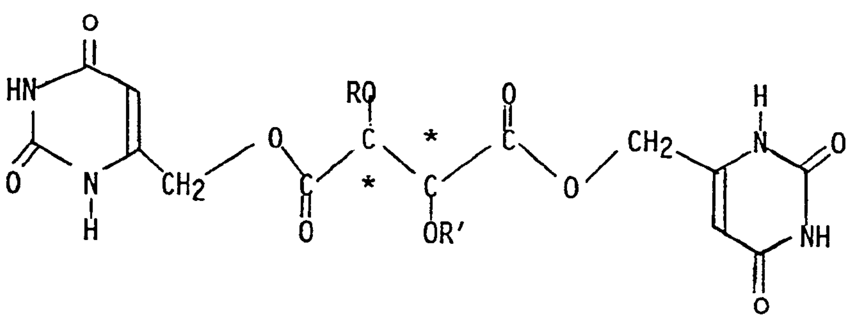

(VII)

S'agissant de M2 :

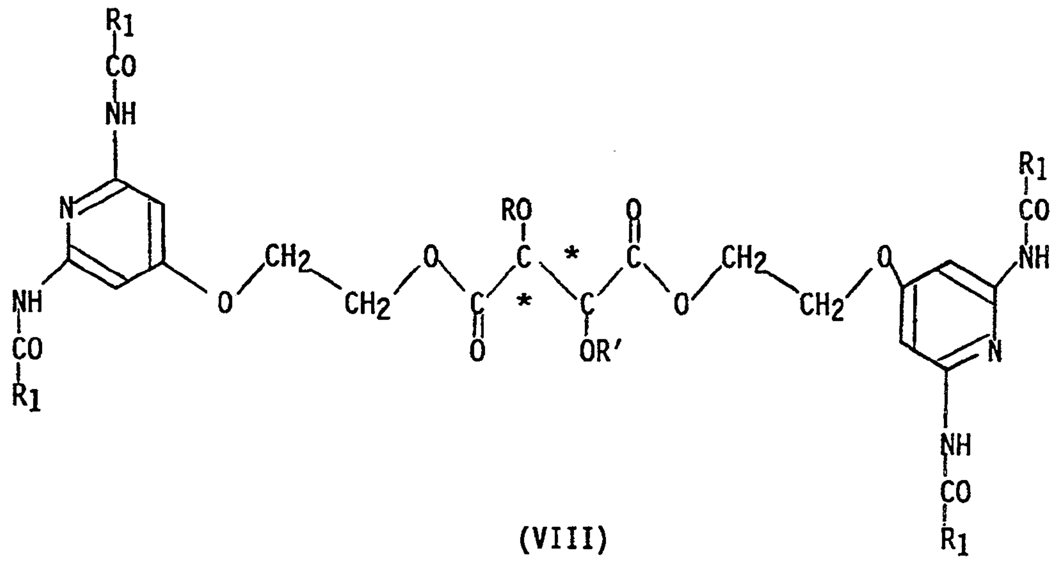

(VIII)

dans lesquelles les symboles R et R′ ont les significations données ci-avant propos de la formule (VI) et le symbole $R_1$ représente un radical alkyle linéaire ayant de 1 à 4 atomes de carbone.

Dans le cadre de ces exemples spécifiques les motifs d'association $\Sigma - \overline{\Sigma}$ des monomères de base présentent la structure, avec trois liaisons hydrogène, suivante :

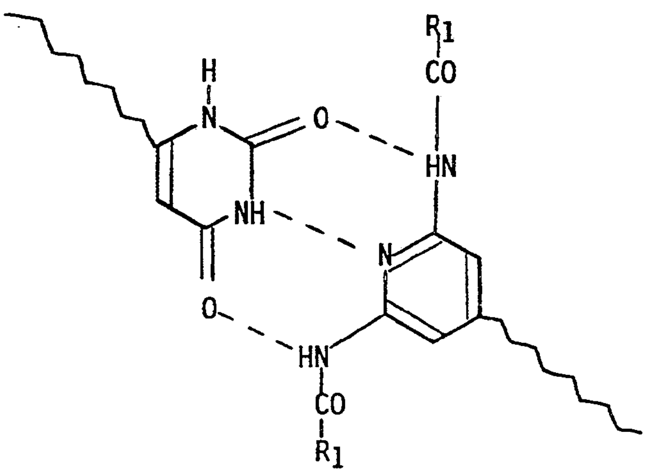

(IX)

La présente invention concerne encore, et c'est ce qui constitue le second objet de la présente invention, un procédé de préparation des polymères dont on vient de parler.

Conformément à ce procédé, il est souhaitable de réaliser un mélange homogène des monomères complémentaires M1 et M2 utilisés généralement en quantités équimolaires. Selon les caractéristiques physiques des ingrédients, cette opération peut consister à appliquer les techniques usuelles pour le mélange de solides finement divisés ou bien à effectuer une solution ou une suspension de l'un des constituants du mélange dans l'autre, maintenu à l'état liquide sous l'effet de la température et/ou par emploi d'un milieu solvant.

De préférence, on opère en présence d'un solvant polaire aprotique ou d'un mélange de pareils solvants commun(s) aux monomères et polymère souhaité en opérant à une température allant de la température ambiante à la température d'ébullition du milieu solvant choisi ; les solvants préférés sont notamment le dioxanne, le tétrahydrofuranne, le chlorure de méthylène, le tétrachlorure de carbone. La réaction de polyassociation est très rapide ; typiquement, on laisse la réaction se dérouler pendant une durée se situant par exemple entre 10 minutes et 2 heures. Le polymère souhaité peut être isolé du (ou des) solvant(s) du mélange réactionnel par exemple par distillation de ce(s) solvant(s) sous pression réduite.

Quand la préparation qui vient d'être décrite se fait en milieu solvant, on doit considérer que la structure du polymère souhaité a atteint sa cohésion maximale une fois le polymère complètement séparé du (ou des) solvant(s) mis en oeuvre.

Les polymères obtenus, en particulier ceux obtenus à partir des monomères M1 et M2 difonctionnels, se

présentent sous la forme d'une pâte opalescente blanchâtre qui possède un caractère collant très prononcé tant vis-à-vis du verre que des surfaces métalliques. Le caractère collant ainsi présenté rend ces polymères utilisables par exemple pour la réalisation de revêtement et de collages. A noter qu'une opération de collage pourrait être réalisée en soudant deux substrats qui ont été traités au préalable l'un par le monomère M1 et l'autre par le monomère M2. L'aptitude que présentent les monomères M1 à reconnaître les monomères M2 pour s'associer par l'intermédiaire de liaisons hydrogène pourrait permettre aussi la réalisation de collages sélectifs d'un substrat donné sur un autre substrat particulièrement choisi. On rappellera que, en jouant sur la nature des radicaux espaceurs A et B, on peut obtenir des polymères thermotropes qui possèdent la qualité de former des phases de cristal liquide (ou mésophase organisée) à des températures de situant dans l'intervalle allant par exemple d'une température inférieure à la température ambiante (25°C) à une température supérieure à 200°C.

La présente invention concerne encore, et c'est ce qui constitue le troisième objet de la présente invention, les monomères de base M1 et M2 de formules (I) et (II) qui sont destinés à la mis en oeuvre du procédé qui vient d'être décrit ; ces monomères constituent des produits nouveaux. La présente invention, dans son troisième objet, concerne en particulier les monomères difonctionnels M1 et M2 de formules (IV) et (V) qui ont été définis ci-avant.

Ces monomères peuvent être préparés à partir de matières premières disponibles en appliquant des méthodes de synthèse connues de l'homme de métier consistant notamment à faire réagir d'une part des précurseurs (des radicaux espaceurs A et B) équipés en bout de chaîne (principale) de deux ou de plus de deux fonctions réactives, comme par exemple les fonctions : acide carboxylique ou un dérivé de cette fonction, alcool, amine primaire ou secondaire, et d'autre part les molécules et groupes de molécules, desquels dérivent les groupes fonctionnels $\Sigma$ et $\bar{\Sigma}$ pourvus de fonctions capables de réagir avec celles portées par les précurseurs des radicaux espaceurs A et B.

Par exemple, les monomères M1 et M2 difonctionnels de formules (VII) et (VIII), dans lesquelles les radicaux espaceurs A et B dérivent de l'acide L(+) tartrique, peuvent être obtenus à partir de L(+)N,N,N',N' tétraméthyltartramide 0 (cf. D. SEEBACH et al. Org. Synth., 1983, 61, 24) en appliquant le schéma de synthèse suivant (dans ce schéma les formules des restes issus de l'acide tartrique sont des formules de projection de NEWMAN) :

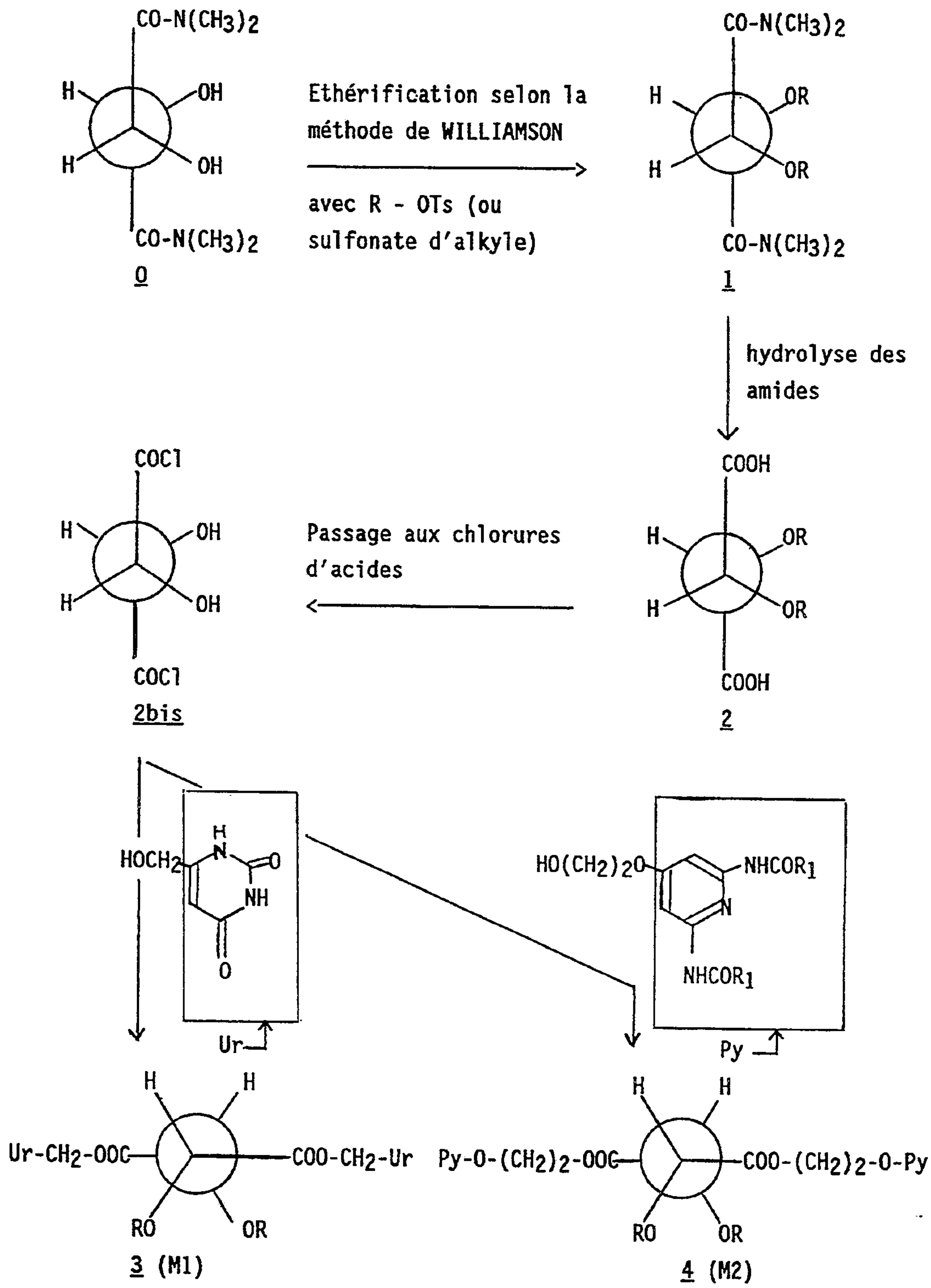


0

1

2bis

2

3 (M1)

4 (M2)

Le réactif $HOCH_2$-Ur ou hydroxyméthyl-6 uracil est préparé selon les indications données par K.L.

NAGPAL, J. Med. Chem., 1972, 15, 121. La préparation du réactif $HO(CH_2)_2O$-Py ou (hydroxyéthyl)oxy-4 diacéthylamino-2,6 pyridine est réalisée en quatre étapes à partir de benzyloxy-4 diamino-2,6 pyridine (cf. D.G. MARKEES et al., J. Med. Chem., 1968, 11, 126) : diacétylation, hydrogénolyse du groupe benzyle, alkylation par le 2-bromoéthylacétate et saponification.

Des monomères M1 et M2 analogues de formules (VII) et (VIII) peuvent être préparés, en suivant le schéma de synthèse précité, à partir de dérivés des acides <u>D(-)</u> et <u>méso</u> tartriques.

La présente invention concerne encore des adducts répondant aux formules suivantes :

$$B'—\bar{\Sigma} \text{ --- } \Sigma—A—\Sigma \text{ --- } \bar{\Sigma}—B' \qquad \text{(X) : adduct A1}$$

$$A'—\Sigma \text{ --- } \bar{\Sigma}—B—\bar{\Sigma} \text{ --- } \Sigma—A' \qquad \text{(XI) : adduct A2}$$

dans lesquelles :

– les ensembles $\Sigma—A—\Sigma$ et $\bar{\Sigma}—B—\bar{\Sigma}$ représentent les monomères difonctionnels M1 et M2 de formule (IV) et (V) qui ont été définis ci-avant, et

– les ensembles $B'—\bar{\Sigma}$ et $A'—\Sigma$ représentent des composés C2 et C1 complémentaires dans la structure desquels : les symboles $\bar{\Sigma}$ et $\Sigma$ représentent les mêmes groupes fonctionnels complémentaires que ceux équipant les monomères M2 et M1 ; les symboles A′ et B′, qui peuvent être identiques ou différents, représentent des substitutions portées par les groupes fonctionnels $\Sigma$ et $\bar{\Sigma}$ et consistent dans de longues chaînes hydrocarbonées, linéaires ou ramifiées, pouvant renfermer un ou plusieurs hétéroatomes.

Comme adducts préférentiellement représentatifs, on peut citer les adducts de formule (X) ou (XI) dans la structure desquels :

(i) l'ensemble $\Sigma—A—\Sigma$ et le groupe fonctionnel complémentaire $\bar{\Sigma}$ ou l'ensemble $\bar{\Sigma}—B—\bar{\Sigma}$ et le groupe fonctionnel complémentaire $\Sigma$ possèdent les définitions données ci-avant à propos des monomères difonctionnels M1 et M2 dénommés "préférentiellement représentatifs" (cf. page 4), et

(2i) la substitution B′ ou A′ est située, dans les composés $B'—\bar{\Sigma}$ ou $A'—\Sigma$, en une position opposée ou sensiblement opposée par rapport à celle occupée par les sites donneur(s) et/ou accepteur du groupe fonctionnel.

Comme adducts plus préférentiellement représentatifs, on peut citer les adducts de formule (X) ou (XI) dans la structure desquels :

(3i) l'ensemble $\Sigma—A—\Sigma$ et le groupe fonctionnel complémentaire $\bar{\Sigma}$ ou l'ensemble $\bar{\Sigma}—B—\bar{\Sigma}$ et le groupe fonctionnel complémentaire $\Sigma$ possèdent les définitions données ci-avant à propos des monomères difonctionnels M1 et M2 dénommés "plus préférentiellement représentatifs" (cf. page 5), et

(4i) la substitution B′ ou A′ (identique ou différente de B′), située en position opposée ou sensiblement opposée par rapport à celle occupée par les sites donneur(s) et/ou accepteur(s), représente une longue chaîne aliphatique hydrocarbonée, pouvant renfermer un ou plusieurs hétéroatome(s) tels que notamment N, O, S, qui est ramifiée et répond aux conditions supplémentaires suivantes :

- la chaîne principale de ladite chaîne ramifiée comporte un nombre total d'atomes n1 se situant dans l'intervalle allant de 15 à 25,
- ladite chaîne principale possède une chaîne secondaire comportant un nombre d'atomes n2 se situant dans l'intervalle allant de 11 à 20,
- la somme des nombres d'atomes n1 + n2 au niveau du substituant B′ ou A′ est au moins égal à 28 atomes.

Comme adducts encore plus préférentiellement représentatifs, on peut citer les adducts de formule (X) ou (XI) dans la structure desquels :

(5i) l'ensemble $\Sigma—A—\Sigma$ et le groupe fonctionnel complémentaire $\bar{\Sigma}$ ou l'ensemble $\bar{\Sigma}—B—\bar{\Sigma}$ et le groupe fonctionnel complémentaire $\Sigma$ possèdent les définitions données ci-avant à propos des monomères difonctionnels M1 et M2 dénommés "encore plus préférentiellement représentatifs" (cf. page 6), et

(6i) la substitution B′ ou A′ a la définition (4i) donnée ci-avant.

A titre d'exemples spécifiques d'adducts qui appartiennent au groupe des adducts "encore plus préférentiellement représentatifs", on citera notamment les adducts A1 et A2 obtenus à partir :

– s'agissant de A1 : du mélange du monomère M1 de formule (VII) et du composé complémentaire C2 (à raison de 2 moles par mole de M1) de structure :

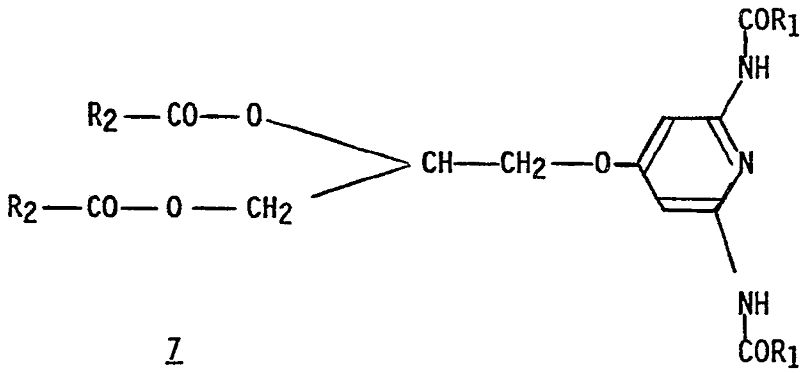

7

dans laquelle : le symbole $R_1$ représente un radical alkyle linéaire ayant de 1 à 4 atomes de carbone ; et le symbole $R_2$ représente un radical alkyle linéaire ayant de 11 à 18 atomes de carbone ;
– s'agissant de A2 : du mélange du monomère M2 de formule (VIII) et du composé complémentaire C1 (à raison de 2 moles par mole de M2) de structure :

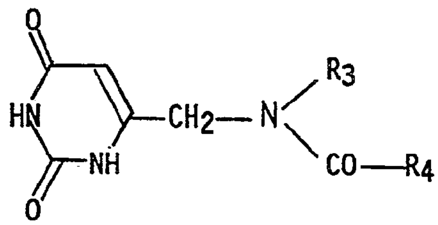

11

dans laquelles : le symbole $R_3$ représente un radical alkyle linéaire ayant de 12 à 20 atomes de carbone; le symbole $R_4$, identique à/ou différent de $R_3$, représente un radical alkyle linéaire ayant de 11 à 18 atomes de carbone ; avec la condition supplémentaire selon laquelle la somme des atomes de carbone contenus dans l'ensemble $R_3$ + $R_4$ est égal au moins à 25.

Le composé C2 correspond à des espèces chimiques qui peuvent être obtenues à partir d'allyloxy-4 diamino-2,6 pyridine 5 (cf. D.G. MARKEES et al., J. Am. Chem. Soc., 1956, 78, 4130) en appliquant le schéma de synthèse suivant :

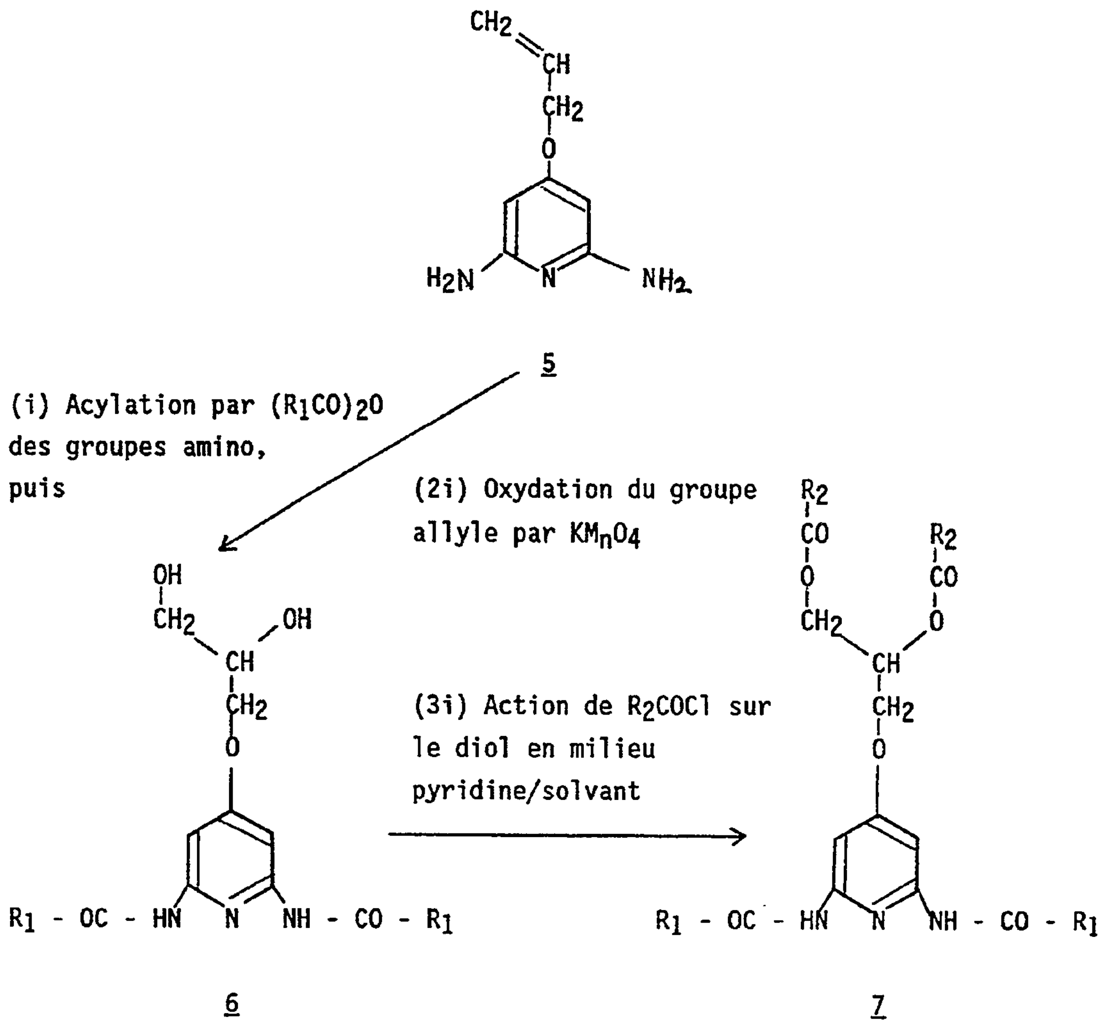


5

6

7

Le composé C1 correspond à des espèces chimiques qui peuvent être obtenues à partir de formyl-6 uracil 8 (cf. T.B. JOHNSON et al., J. Am. Chem. Soc., 1931, 53, 1989) ou de chlorométhyl-6 uracil 10 (cf. I. VON KLOSA, Arzneim. Forsch/Drug Res., 1980, 30, 228) en appliquant le schéma de synthèse suivant :

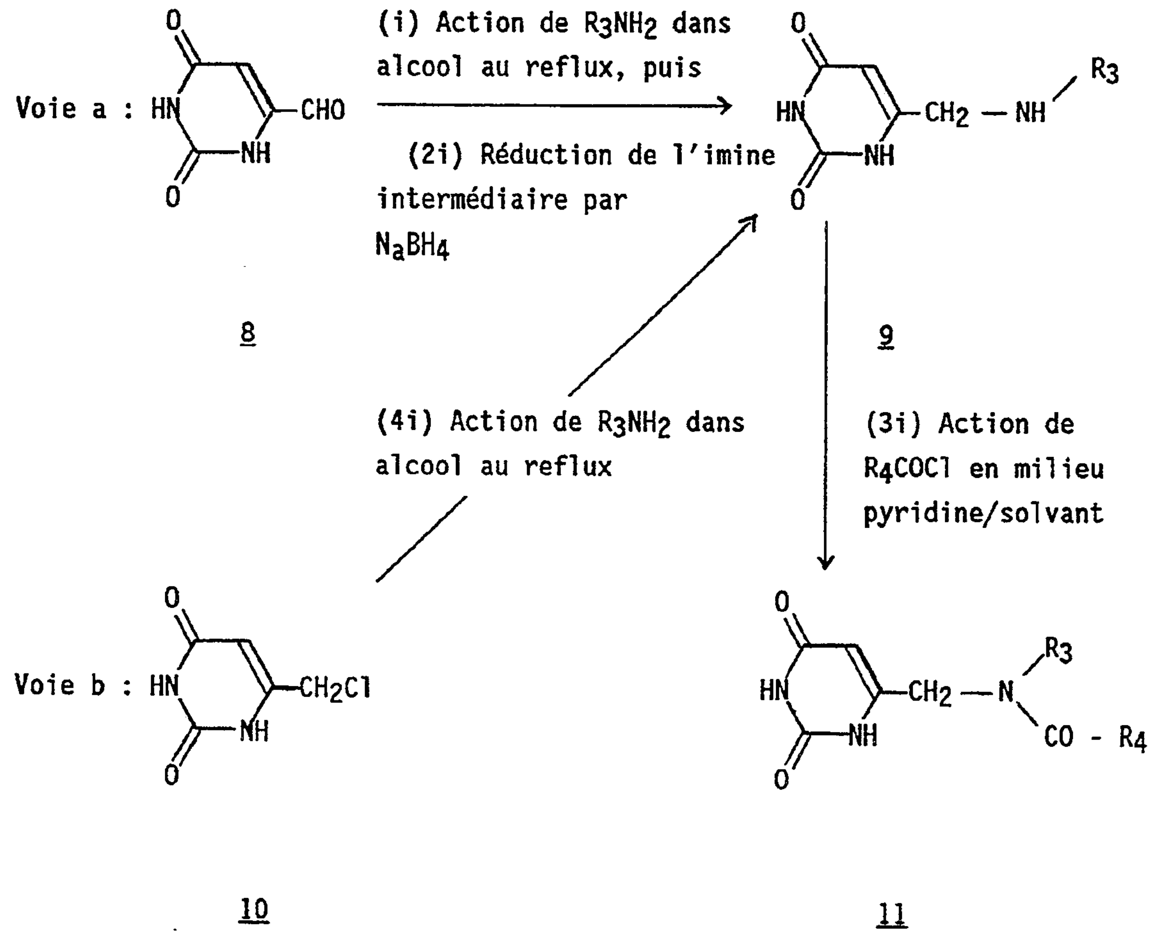


Les exemples qui suivent illustrent de manière non limitative comment la présente invention peut être mise en pratique.

Dans ces exemples, les polymères conformes à la présente invention, ainsi que les monomères complémentaires qui leur donnent naissance, ont été identifiés par différentes analyses concernant la détermination des caractéristiques de la structure chimique (infra-rouge ; résonance magnétique nucléaire du proton et du carbone 13 ; analyse élémentaire). Les points de fusion et les caractéristiques des propriétés thermotropes, quand il y en a, ont été déterminées : par analyse calorimétrique différentielle (DSC) à l'aide d'un appareil PERKIN-ELMER DSC 2 équipé d'un calculateur HEWLETT-PACKARD HP 86 pour l'acquisition et le traitement des données ; par des observations au moyen d'un microscope polarisant couplé à une platine chauffante qui permet d'identifier la nature des phases en présence ; et par des examens par diffraction des rayons X (RX). Les pouvoirs rotatoires ont été mesurés sur un polarimètre PERKIN-ELMER 241.

EXEMPLE 1 :

On décrit dans cet exemple la préparation et les caractéristiques du monomère M1 de formule (VII) dans laquelle :

– le radical espaceur A dérive de l'acide L(+)tartrique, et
– les radicaux R et R' des restes éther portés par les carbones asymétriques sont identiques et représentant chacun le radical $C_{12}H_{25}$.

Cette préparation est réalisée en suivant le schéma de synthèse présenté ci-avant à la page 11 et le monomère M1 obtenu ici est désigné par la notation 3a (structure 3 avec R = $C_{12}H_{25}$).

L(+)didodécyloxy-N,N,N',N'-tétraméthyltartramide ; structure 1 avec R = $C_{12}H_{25}$ notée 1a :

Dans un ballon tricol de 250 ml balayé par un courant d'azote, on introduit 10 mmoles (480 mg) de NaH (suspension huileuse à 50%) qu'on lave trois fois au pentane sec. On ajoute 18 ml de diméthylformamide (DMF)

anhydre, puis, par petites portions, 5 mmoles (1,02 g) de L(+)N,N,N',N'-tétraméthyltartramide O. Il se forme un gel épais qui mousse avec le dégagement d'hydrogène. On agite 1 heure à température ambiante sous azote, puis on ajoute 10 mmoles de p-toluènesulfonate de n-dodécyle (4,6 g) en solution dans 4 ml de DMF, et on chauffe à 80°C pendant 8 heures (le gel devient rapidement une solution homogène). Après refroidissement et addition d'eau, on extrait à l'éther, lave avec de la saumure, sèche sur $Na_2SO_4$ et évapore à sec. Le produit brut est purifié par "chromatoflash" sur silice Amicon, élué par le mélange dichlorométhane ($CH_2Cl_2$)/acétone 3/1.

Rendement 80% ; cristallise dans l'hexane, F < 50°C ; $[\alpha]_D^{25}$ = +45° (c = 0,1 $CHCL_3$).

$^1H$ RMN ($CHCl_3$) : $\underline{CH_3}$ - $CH_2$ -:0,89, t., (6H) ; $\underline{CH_3}$-N:2,92, s., (6H); $\underline{CH_3}$-N:3,18, s., (6H) ; R-$\underline{CH_2}$-O-:3,55, t., (4H) ; -O-$\underline{CH}$-$CO_2H$:4,69, s., (2H).

Acide L(+)didodécyloxytartrique ; structure 2 avec R = $C_{12}H_{25}$ notée 2a :

L'hydrolyse de l'amide 1a est effectuée selon T.M. FYLES et al., Can. J. Chem., 1984, 62, 498 : deux mmoles d'amide et 15 ml de HCl 25 % sont chauffés à reflux pendant 15 heures. Après refroidissement et dilution, on essore les cristaux formés.

Rendement quantitatif ; cristallise dans l'hexane ; F = 76°C ; $[\alpha]_D^{25}$ = +14,5° (c = 0,1, $CHCl_3$).

$^1H$ RMN ($CDCl_3$):$\underline{CH_3}$-$CH_2$-:0,87, t., (6H); R-$\underline{CH_2}$-O-:3,5,q., (2H) et 3,72, q., (2H) ; -O-$\underline{CH}$-$CO_2H$:4,36, s., (2H) ; -$CO_2\underline{H}$:9,7, s.l. (2H).

L(+)didodécyloxytartrate de 6-hydroxyméthyluracile 3a :

a) Passage au chlorure d'acide 2bisa :

A une solution de 1 mmole de diacide 2a (486 mg) dans 3 ml de $CH_2Cl_2$ anhydre, on ajoute par portions 2 mmoles (416 mg) de $PCl_5$, et on laisse réagir 18 heures à température ambiante. On évapore sous vide trois fois en présence de toluène, puis le résidu est dissous dans 3 ml de $CH_2Cl_2$ sec.

b) Estérification

Cette solution est ajoutée, sous azote et à -10°C, à une solution de 2 mmoles (284 mg) de 6-hydroxyméthyluracil et 2,2 mmoles (268 mg) de diméthylaminopyridine (DMAP) dans 2 ml d'hexaméthylphosphotriamide (HMPT) anhydre. On agite 3 heures à température ambiante, puis on ajoute de l'eau.

Le mélange est extrait une fois à l'acétate d'éthyle ; l'ester 3a, pratiquement pur, en suspension dans la partie aqueuse est essoré ; rendement : 54%. Il est purifié par cristallisation dans l'éthanol et chromatographie sur couche mince des eaux-mères (plaques Macherey-Nagel de gel de silice, THF/$CH_2Cl_2$ 1/1).

Analyse (désolvaté) : $C_{38}H_{62}N_4O_{10}$ (M = 734,91), calculé : C % 62,10, H % 8,50, N % 7,68 ; trouvé : C % 62,58, H % 8,65, N % 7,86.

$[\alpha]_D^{27}$ = +37°, $[\alpha]_{546}^{27}$ = +44,5° (c = 0,55, THF).

Les propriétés physiques (RMN, points de fusion) sont rassemblées dans les tableaux 1 et 2 suivants.

EXEMPLE 2 :

On décrit dans cet exemple la préparation et les caractéristiques du monomère M2 de formule (VIII) dans laquelle :

- les radicaux R1 représentent chacun $CH_3$,
- le radical espaceur B dérive de l'acide L(+)tartrique, et
- les radicaux R et R' des restes éther portés par les carbones asymétriques sont identiques et représentent chacun le radical $C_{12}H_{25}$.

Cette préparation est réalisée en suivant le schéma de synthèse présenté ci-avant à la page 11 et le monomère M2 obtenu ici est désigné par la notation 4a (structure 4 avec R = $C_{12}H_{25}$).

Le L(+)didodécyloxytartrate de 2,6-diacétylamino-4-(hydoxyéthyl)oxy-pyridine 4a est préparé à partir du diacide 2a obtenu comme indiqué ci-avant dans l'exemple 1.

La préparation des chlorures d'acides et leur estérification par le 2,6-diacétylamino-4-(hydroxyéthyl)oxy-pyridine sont effectuées dans les mêmes conditions que celles mentionnées ci-avant dans l'exemple 1 à propos de l'ester 3a, en remplaçant toutefois le HMPT par le DMF.

Après addition d'eau,le mélange réactionnel est extrait au dichlorométhane et le produit brut est purifié par chromatographies successives sur colonne (silice Merck) ou sur plaques (Macherey-Nagel), en éluant par le mélange dichlorométhane/éthanol 85/15. On recueille 0,29 mmole (278 mg) d'ester 4a ; rendement = 29 %. Ce dernier cristallise en se solvatant dans l'éthanol aqueux.

Analyse (désolvaté à température ambiante) : $C_{50}H_{80}N_6O_{12}$ (M = 957,23) :

| | | | | |
|---|---|---|---|---|
| - calculé | : | C % 62,74 | H % 8,42 | N % 8,78 |
| + 1/2 $H_2O$ | : | 62,15 | 8,45 | 8,70 |
| - trouvé | : | 62,05 | 8,60 | 8,64 |

$[\alpha]^{27}_{D}$ = +25,5°, $[\alpha]^{27}_{546}$ = +30°(c = 0,5, THF).

Les propriétés physiques (RMN, points de fusion) sont rassemblées dans les tableaux 1 et 2 suivants :

EXEMPLES 3 ET 4 :

On décrit dans ces exemples les monomères M1 (exemple 3) et M2 (exemple 4) possédant des structures du même type que celles des monomères obtenus dans les exemples 1 et 2 précédents, mais dans lesquels les radicaux espaceurs A et B dérivent maintenant de l'acide mésotartrique, lequel possède en projection de NEWMAN la formule suivante :

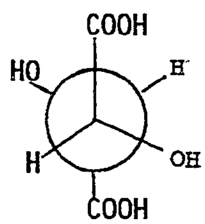

Ces monomères sont désignés par les nouvelles notations 13a (M1) et 14a (M2).

Les dérivés de l'acide mésotartrique, qui apparaissent ci-après, ont été préparés à partir du méso-N,N,N',N'-tétraméthyltartramide exactement dans les mêmes conditions que celles décrites ci-avant dans les exemples 1 et 2 pour la série L(+). Leurs caractéristiques sont les suivantes :

Mésodidodécyloxy N,N,N',N'-téraméthyltartramide :

Rendement 51 % ; F = 79°C ; $^1H$ RMN ($CDCl_3$) : -O-CH-$CO_2H$ : 4,52 s. (2H) ; $-CH_2$-O- : 3,4 m. (4H) ; $(CH_3)_2N$- : 3,13 s. (6H) et 3,0 s. (6H).

Acide mésodidodécyloxytartrique

Rendement 100 % ; F = 84-88°C ; $^1H$ RMN ($CDCl_3$) : $-CO_2H$ : 9,45 s. l. (2H) ; -O-CH-$CO_2H$ : 4,38 s. (2H) ; $-CH_2$-O ; 3,7 m. (4H).

Mésodidodécyloxytartrate de 6-hydroxyméthyluracil 13a :

Rendement 33% : RMN et fusion : voir tableaux 1 et 2 suivants.

Analyses : $C_{38}H_{62}N_4O_{10}$ (M = 734,91) calculé : C % 62,10, H % 8,50 N % 7,62 ; trouvé : C % 62,02, H % 8,46, N % 7,76.

Mésodidodécyloxytartrate de 2,6-diacétylamino-4-(hydroxyéthyl)oxy-pyridine 14a :

Rendement 36% : RMN et fusion : voir tableaux 1 et 2.
Analyses : $C_{50}H_{80}N_8O_{10}$ (M = 957,23) calculé : C % 62,74, H % 8,42, N % 8,78 ; trouvé : C % 62,90, H % 8,47, N % 8,92.

EXEMPLES 5 ET 6 :

On décrit dans ces exemples deux polymères conformes à la présente invention qui sont préparés en dissolvant dans le tétrahydrofuranne (THF) des quantités exactement équimolaires de monomères complémentaires M1 et M2. Les solutions ont été ensuite évaporées et le résidu séché pendant 20 minutes à 80°C sous $20.10^2$ Pa.

1) Exemple 5 : mélange M1 + M2 de composition : 3a + 4a.

Le polymère obtenu se présente comme une glu épaisse, collant au verre et à la spatule, très biréfringeante, fondant à 254°C, soit 34° et 196° plus haut que ses composants. Il est soluble dans le THF, moins soluble dans le chloroforme, quasiment insoluble dans l'éthanol ; en concentrant les solutions (THF et $CHCl_3$), on obtient des gels qui donnent des textures visibles au microscope polarisant telles que notamment des structures en bandes semblables à celles de polymères thermotropes.

Analyse (sans désolvatation) : $C_{88}H_{142}H_{10}O_{22}$ (M = 1692,14) : calculé : C % 62,46, H % 8,46, N % 8,28 ; trouvé : C % 62,45, H % 8,53, N % 8,16.

La RMN et les températures de transition sont donnés dans les tableaux 1 et 2 suivants.

L'analyse aux RX du polymère obtenu montre que la mésophase est de type colonnaire hexagonal (diamètre de colonne = 38,5 Å), chaque colonne étant formée par trois brins résultant de l'association des deux monomères complémentaires sous forme de polymère. Les données sont compatibles avec un modèle moléculaire comportant trois brins équivalents enroulés sur une hélice de pas = 12,8 Å et de rayon = 7,35 Å ; la symétrie de la colonne est ternaire et on peut placer 3,71 motifs d'association du type $\Sigma — \overline{\Sigma}$ sur un tour (120° par brin).

2) Exemple 6 : mélange M1 + M2 de composition : 13a + 14a

Le polymère obtenu ici est également un composé défini, dont les propriétés sont analogues à celles du polymère 3a + 4a décrit plus haut, avec cependant de nettes différences.

La RMN et les températures de transition sont données dans les tableaux 1 et 2 suivants.

Les solubilités sont à peu près les mêmes et des gels se forment également en concentrant les solutions, mais les textures observées sous microscope sont nettement différentes ; comme ci-dessus, les rayons X révèlent une mésophase colonnaire, avec des colonnes de diamètre = 36,8 Å possédant une symétrie ternaire, mais où les trois brins assemblés dans une colonne se disposent en zigzag et non en hélice ; on peut placer 3 motifs du type $\Sigma — \overline{\Sigma}$ sur un tour.

TABLEAU 1 : $^1H$ RMN (200 MHz)

| EXEMPLE | NH | H aromatique | C = C - H | $O-CH_2-Ur$ |
|---|---|---|---|---|
| 1 : 3a | 10,32 s.l.*<br>10,06 s.l. | - | 5,71 s. | 5,03 s. |
| 2 : 4a | 9,14 s. | 7,74 s. | - | - |
| 5 : 3a+4a<br>(THF d8) | 11,11 s.l.<br>9,29 s.l.<br>8,04 s.l. | 7,74 s. | 5,75 s. | 5,03 s. |
| 3 : 13a<br>($CDCl_3$-DMSOd6)<br>** | 10,9 s.<br>10,82 s. | - | 5,5 s. | 4,76 s. |
| 4 : 14a<br>($CDCl_3$) | 7,91 s.l. | 7,36 s. | 5,52 s. | - |
| 6 : 13a+14a<br>(DMSO d6) | 11,05 s.l.<br>9,99 s.l. | 7,36 s. | 5,52 s. | 4,8 s. |

| EXEMPLE | $-O-\underset{\vert}{C}H-CO_2$ | $O-CH_2-CH_2-O$ | $R-CH_2-O$ | $CH_3CO$ |
|---|---|---|---|---|
| 1 : 3a | 4,69 s. | - | 3,90 q.<br>3,57 q. | - |
| 2 : 4a | 4,53 s. | 4,59 q.<br>4,42 q. | 3,85 q.<br>3,47 q. | 2,22 s. |
| 5 : 3a+4a<br>(THF d8) | 4,69 s.<br>4,52 s. | 4,59 m.<br>4,41 m. | sous THF<br>3,51 | 2,22 s. |
| 3 : 13a<br>($CDCl_3$-DMSOd6) | 4,30 s. | - | 3,59 q.<br>3,40 q. | - |
| 4 : 14a<br>($CDCl_3$) | 4,45 s. | 4,44 s.l.<br>4,19 s.l. | 3,63 q.<br>3,46 q. | 2,15 s. |
| 6 : 13a+14a<br>(DMSO d6) | 4,45 s. | 4,40 s.l.<br>4,14 s.l. | sous $H_2O$ | 2,06 s. |

* l. = large          ** = dimétylsulfoxyde

TABLEAU 2

Températures en °C et enthalpies de transition ΔH en $kcal.mol^{-1}$ des monomères complémentaires et des polymères formés

| EXEMPLE | COMPOSE | TRANSITION a) | |
|---|---|---|---|
| | | T(°C) | ΔH |
| 1 | monomère M1 : 3a | K 216-220 I | 11 |
| 2 | monomère M2 : 4a | K 29 ; 45 ; 58 I | 3 |
| 5 | polymère : 3a+4a | K < 25 M 254 I | 35 (M —>I) |
| 3 | monomère M1 : 13a | K 190-193 I | - |
| 4 | monomère M2 : 14a | K 109-115 I | 8,2 |
| 6 | polymère : 13a+14a | K < 25 M 219-222 I | 22,5 |

a) K = phase cristalline ; M = phase mésomorphe ; I = phase isotrope. Les échantillons sont examinés au chauffage puis au refroidissement sur au moins deux cycles (vitesses 5 ou 10°C $min^{-1}$). Les températures de transition correspondent soit à l'intersection entre la ligne de base et la tangente à la plus grande pente du pic (pic fin) soit au maximum du pic (pic large). Les enthalpies de transition sont déduites de la surface des pics en prenant l'indium comme étalon (enthalpie de fusion 785 $cal.mol^{-1}$).

EXEMPLE 7 :

On décrit dans cet exemple la préparation et les propriétés physiques d'un adduct A1 obtenu à partir du mélange du monomère M1 de structure 3a décrit dans l'exemple 1 et du composé complémentaire C2 (à raison de 2 moles par mole de M1) de structure 7 (cf. ci-avant page 14) dans laquelle $R_1 = CH_3$ et $R_2 = C_{17}H_{35}$, notée 7b.

Le mélange a été préparé par dissolution à chaud dans le THF, suivi de l'évaporation puis du séchage pendant une heure. Les températures et enthalpies de transition [ΔH] en $Kcal.mol^{-1}$ mesurées par DSC sont les suivantes :

K 34-39 M 215 - 218 [33,6] I

L'adduct obtenu est très visqueux, mais s'oriente très bien par frottement unidirectionnel.

**Revendications**

1. Polymères éventuellement thermotropes caractérisés en ce qu'ils résultent de l'interaction de :
   a) un monomère M1 de formule :

$$\left[\Sigma -\right]_{m1} A \qquad (I)$$

dans laquelle :

- les symboles Σ, identiques ou différents entre eux, représentent chacun un groupe fonctionnel, dérivant d'une molécule ou d'un groupe de molécules, qui est capable de former au moins deux liaisons hydrogène avec le groupe fonctionnel complémentaire représenté par chacun des symboles $\bar{\Sigma}$ du monomère M2 de formule (II) apparaissant ci-après,
- le symbole A représente un radical organique de valence ml pouvant renfermer jusqu'à 50 atomes de carbone, et
- ml représente un nombre au moins égal à 2 ;

avec b) un monomère complémentaire M2 de formule :

$$\left[\bar{\Sigma} -\right]_{m2} B \qquad (II)$$

dans laquelle :

- les symboles $\bar{\Sigma}$ identiques ou différents entre eux, représentent chacun un groupe fonctionnel, dérivant d'une molécule ou d'un groupe de molécules, qui est capable de former au moins deux liaisons hydrogène avec le groupe fonctionnel complémentaire représenté par chacun des symboles Σ du monomère M1 de formule (I) défini ci-avant,
- le symbole B représente un radical organique de valence m2, identique ou différent du radical A, et
- m2 représente un nombre au moins égal à 2.

**2.** Polymères selon la revendication 1, caractérisés en ce que les groupes fonctionnels Σ et $\bar{\Sigma}$ qui doivent comprendre chacun au moins deux sites donneur(s) et/ou accepteur(s), dérivent de molécules ou groupes de molécules consistant dans des composés monocycliques contenant un ou plusieurs hétéroatome(s) tels que notamment N, O, S, ou dans des composés polycycliques, ortho- ou péricondensés, contenant également un ou plusieurs hétéroatome(s) tels que notamment N, O, S, les sites donneurs et accepteurs capables de former des liaisons hydrogène pouvant (i) être contenus dans le (ou les) cycle(s) et/ou (2i) constituer une (ou plusieurs) substitution(s) portée(s) par ce (ou ces) cycle(s).

**3.** Polymères selon la revendication 1, caractérisés en ce qu'ils sont obtenus à partir de monomères M1 et M2 difonctionnels de formules :

$$\underset{M1}{\Sigma - A - \Sigma} \quad (IV) \qquad \text{et} \qquad \underset{M2}{\bar{\Sigma} - B - \bar{\Sigma}} \quad (V)$$

dans lesquelles :

- les groupes fonctionnels complémentaires Σ (identiques ou différents entre eux) et $\bar{\Sigma}$ (identiques ou différents entre eux), qui doivent comprendre chacun au moins deux sites donneur(s) et/ou accepteur(s), dérivent de molécules ou groupes de molécules consistant dans des composés monocycliques contenant un ou plusieurs hétéroatome(s) tels que notamment N, O, S ou dans des composés polycycliques, ortho- ou péricondensés, contenant également un ou plusieurs hétéroatome(s) tels que notamment N, O, S, les sites donneurs et accepteurs capables de former des liaisons hydrogène pouvant (i) être contenus dans le (ou les) cycle(s) et/ou (2i) constituer une (ou plusieurs) substitution(s) portée(s) par ce (ou ces) cycle(s),
- les radicaux espaceurs A et B, identiques ou différents, représentent des radicaux divalents consistant dans des chaînes hydrocarbonées, linéaires ou ramifiées, saturées ou insaturées, comprenant éventuellement un ou plusieurs hétéroatomes tels que notamment N, O, S, qui comportent au moins

2 atomes de carbone dans la chaîne principale, ladite chaîne principale étant celle qui porte en bout de chaîne les deux groupes fonctionnels Σ et $\overline{\Sigma}$.

4. Polymères selon la revendication 3, caractérisés en ce que les monomères M1 et M2 utilisables sont des composés de formules (IV) et (V) dans lesquelles :
   – les groupes fonctionnels complémentaires Σ (identiques ou différents entre eux) et $\overline{\Sigma}$ (identiques ou différents entre eux) dérivent des couples de composés hétérocycliques complémentaires suivants : c1 = cytosine/isocytosine, c2 = guanine/cytosine, c3 = adénine/uracil, c4 = dialkanamido-2,6 pyridine/uracil, c5 = dialkanamido-2,6 pyridine/thymine,
   – les radicaux espaceurs A et B sont fixés sur les groupes fonctionnels en des points situés en une position opposée ou sensiblement opposée par rapport à celle occupée par les sites donneur(s) et/ou accepteur(s).

5. Polymères selon la revendication 3, caractérisés en ce que les monomères M1 et M2 utilisables sont des composés de formules (IV) et (V) dans lesquelles :
   – les paires de groupes fonctionnels complémentaires sont formées chacune de deux groupes Σ identiques et de deux groupes $\overline{\Sigma}$ identiques,
   – les groupes fonctionnels complémentaires dérivent des couples de composés hétérocycliques complémentaires suivants : c1 = cytosine/isocytosine, c2 = guanine/cytosine, c3 = adénine/uracil, c4 = dialkanamido-2,6 pyridine/uracil, c5 = dialkanamido-2,6 pyridine/thymine,
   – les radicaux espaceurs A et B sont fixés sur les groupes fonctionnels en des points situés en une position opposée ou sensiblement opposée par rapport à celle occupée par les sites donneur(s) et/ou accepteur(s),
   – les radicaux espaceurs A et B, identiques ou différents, représentent des radicaux divalents consistant dans des chaînes aliphatiques saturées, linéaires ou ramifiées, comprenant éventuellement un ou plusieurs hétéroatomes tels que notamment N, O, S, qui comportent au moins 6 atomes dans la chaîne principale dont au moins 4 atomes de carbone.

6. Polymères thermotropes selon l'une quelconque des revendications 3 à 5, caractérisés en ce que les monomères M1 et M2 utilisables présentent une structure dans laquelle les radicaux espaceurs A et B, identiques ou différents, représentent tous deux des radicaux divalents consistant dans des chaînes ramifiées, comprenant éventuellement un ou plusieurs hétéroatomes, qui répondent aux conditions supplémentaires suivantes :
   • la chaîne principle de chaque chaîne ramifiée, qui est celle portant en bout de chaîne les deux groupes fonctionnels Σ et $\overline{\Sigma}$, comporte au moins 6 atomes dont au moins 4 atomes de carbone, et
   • la (ou les) chaîne(s) latérale(s) attachée(s) à ladite chaîne principale comporte(nt) au moins 6 atomes dont au moins 5 atomes de carbone.

7. Polymères thermotropes selon la revendication 3, caractérisés en ce que les monomères M1 et M2 utilisables sont des composés de formule (IV) et (V) dans lesquelles :
   – les paires de groupes fonctionnels complémentaires sont formées chacune de deux groupes Σ identiques et de deux groupes $\overline{\Sigma}$ identiques,
   – les groupes fonctionnels complémentaires dérivent des couples de composés hétérocycliques complémentaires suivants : c1 = cytosine/isocytosine, c2 = guanine/cytosine, c3 = adénine/uracil, c4 = dialkanamido-2,6 pyridine/uracil, c5 = dialkanamido-2,6 pyridine/thymine,
   – les radicaux espaceurs A et B sont fixés sur les groupes fonctionnels en des points situés en une position opposée ou sensiblement opposée par rapport à celle occupée par les sites donneur(s) et/ou accepteur(s),
   – les radicaux espaceurs A et B, identiques ou différents, dérivent chacun de l'acide tartrique, pris dans sa configuration méso ou dans ses configurations thréo, et correspondent chacun à la formule :

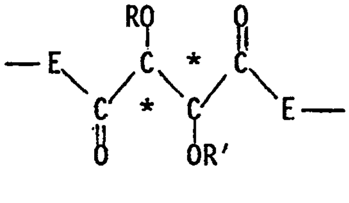

(VI)

dans laquelle :

- les restes E, identiques ou différents, représentent chacun un motif ayant au moins un atome, choisi dans le groupe des atomes de carbone et des hétéroatomes tels que notamment N, O, S, résultant de l'accrochage de la fonction COOH (ou d'un dérivé de cette fonction) de l'acide tartrique sur une fonction réactive appropriée attachée soit directement par un lien de covalence, soit par l'intermédiaire d'un bras d'espacement à base d'atome(s) de carbone et/ou d'hétéroatome(s) sur le groupe fonctionnel $\Sigma$ ou $\bar{\Sigma}$,
- les symboles R et R', identiques ou différents, représentent chacun un radical alkyle linéaire ayant de 5 à 20 atomes de carbone.

**8.** Procédé de préparation des polymères selon l'une quelconque des revendications 1 à 7, caractérisé en ce qu'il consiste à réaliser, par tous moyens connus en soi, un mélange homogène des monomères complémentaires M1 et M2 qui sont engagés en quantités équimolaires.

**9.** Procédé selon la revendication 8, caractérisé en ce qu'il est conduit en présence d'un solvant polaire aprotique ou d'un mélange de pareils solvants commun(s) aux monomères et polymère souhaité en opérant à une température allant de la température ambiante à la température d'ébullition du milieu solvant choisi.

**10.** A titre de produits nouveaux spécialement destinés à la mise en oeuvre du procédé selon l'une quelconque des revendications 8 et 9, les monomères M1 et M2 de formule :

$$M1 : \left[\Sigma\right]_{m1}\!\!-\!\!-\!\!-A \qquad (I)$$

$$M2 : \left[\bar{\Sigma}\right]_{m2}\!\!-\!\!-\!\!-B \qquad (II)$$

dans lesquelles les symboles $\Sigma$, $\bar{\Sigma}$, A, B, m1 et m2 ont les significations données ci-avant dans la revendication 1.

**11.** Produits selon la revendication 10, caractérisés en ce qu'il s'agit de composés difonctionnels de formules:

$$M1 : \Sigma - A - \Sigma \quad (IV)$$

$$M2 : \bar{\Sigma} - B - \bar{\Sigma} \quad (V)$$

dans lesquelles les symboles $\Sigma$, $\bar{\Sigma}$, A et B ont les significations données ci-avant dans l'une quelconque des revendications 3 à 7.

**12.** A titre de produits nouveaux des adducts répondant aux formules suivantes :

$$B' - \bar{\Sigma}\ \text{---}\ \Sigma - A - \Sigma\ \text{---}\ \bar{\Sigma} - B' \qquad (X)\ :\ \text{adduct A1}$$

$$A' - \Sigma\ \text{---}\ \bar{\Sigma} - B - \bar{\Sigma}\ \text{---}\ \Sigma - A' \qquad (XI)\ :\ \text{adduct A2}$$

dans lesquelles :

– les ensembles $\Sigma - A - \Sigma$ et $\bar{\Sigma} - B - \bar{\Sigma}$ représentent les monomères difonctionnels M1 et M2 de formule (IV) et (V) qui ont été définis ci-avant dans la revendication 11, et

– les ensembles $B' - \bar{\Sigma}$ et $A' - \Sigma$ représentent des composés C2 et C1 complémentaires dans la structure desquels : les symboles $\bar{\Sigma}$ et $\Sigma$ représentent les mêmes groupes fonctionnels complémentaires que ceux équipant les monomères M2 et M1 ; les symboles A' et B', qui peuvent être identiques ou différents, représentent des substitutions portées par les groupes fonctionnels $\Sigma$ et $\bar{\Sigma}$ et consistent dans de longues chaînes hydrocarbonées, linéaires ou ramifiées, pouvant renfermer un ou plusieurs hétéroatomes.

Office européen des brevets

# RAPPORT DE RECHERCHE EUROPEENNE

Numero de la demande

EP 90 42 0535

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 066 827 (T. SEITA et al.)<br>* exemples *<br>--- | 1 | C 07 D 239/54<br>C 07 D 213/75<br>C 08 G 81/02<br>C 07 H 19/16<br>C 07 H 21/00 |
| A | SOVIET INVENTIONS ILLUSTRATED semaine 8411, 25 avril 1984, abstract no. 84-067236/11, Derwent Publications Ltd., GB; & SU - A - 1016319 (LENINGRAD LENSOVET TECH) 07.05.1983<br>----- | 1 | |
| | | | **DOMAINES TECHNIQUES RECHERCHES (Int. Cl.5)** |
| | | | C 07 D 239/00<br>C 07 D 213/00<br>C 08 G 81/00<br>C 07 H 19/00<br>C 07 H 21/00 |

Le présent rapport a été établi pour toutes les revendications

| Lieu de la recherche | Date d'achèvement de la recherche | Examinateur |
|---|---|---|
| BERLIN | 30-01-1991 | VAN AMSTERDAM L.J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)